# EUROPEAN PATENT APPLICATION

(11) **EP 2 745 877 A1**
(43) Date of publication of application: **25.06.2014**
(21) Application number: 12198550.1
(22) Date of filing: 20.12.2012
(51) Int. Cl.: A61Q 11/00, A61K 8/37, A61K 31/23

(54) **Oral care product comprising lactylate**

(71) Applicant: PURAC Biochem BV, 4206 AC Gorinchem (NL)
(72) Inventor: Otto, Roel, 4206 AC Gorinchem (NL); Ramirez, Aldana Mariel, 4206 AC Gorinchem (NL)
(74) Representative: van Wezenbeek, Petrus M.G.F.

(57) **Abstract**

The invention relates to an oral care product comprising a lactylate of formula I: 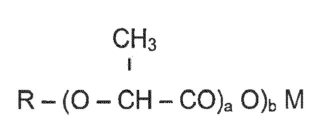 where R = CH₃(CH₂)ₙCO and n is an integer from 4 to 14, M is H⁺, an alkali metal ion, an earth alkali metal ion, or a transition metal ion; b is an integer which corresponds to the valence of M, and a is 1 or 2 , and optionally a surfactant which is not a lactylate having formula I. This invention is targeted to the control of oral bacteria, thereby preventing, reducing, controlling and/or removing dental plaque and oral infections by orally applying the lactylate.

## Description

The invention relates to an oral care product comprising lactylate, to the lactylate per se, and to a method for the control of oral bacteria, thereby preventing, reducing, controlling and/or removing dental plaque and oral infections by orally applying said lactylate.

Although the germicidal activity of fatty acids and derivatives in general has been reported, less is known about the antimicrobial properties of a group of fatty acid derivatives called lactylates.

A lactylate refers to a compound having an acyl group from the fatty acid attached to one (monolactylates) or several lactic acid molecules (dilactylates etc) and a proton (H⁺) or another cation. The fatty acid moiety consists typically of a hydrocarbon chain attached to a carboxyl group at the end. The hydrocarbon chain can contain different numbers of carbon atoms, and the bonds between the carbon atoms can be saturated or unsaturated.

Lactylates are known surfactants. These surfactants are made by reacting lactic acid with fatty acid and neutralizing. Lactylates are well known in the food industry and are used in personal care applications to improve skin feel, skin softness and moisturization and reduce tackiness during wet to dry transition after product application.

Acyl lactylates function as viscosity builders, emulsifiers, foam boosters and stabilizers and can also be used as secondary surfactants.

In US Patent 6,878,757 lactylates are also used as antimicrobial compound in polymeric coatings for sterilized needled sutures. Such lactylates have higher fatty acids esters, particularly stearyl esters such as lithium stearoyl lactylate, potassium stearoyl lactylate, rubidium stearoyl lactylate, cesium stearoyl lactylate, and francium stearoyl lactylate.

WO2009/037270 describes the use of lactylates in nutrition compositions for preventing or treating intestinal infections caused by gram-positive bacteria in animals. In particular, growth of the bacteria of the genus Clostridium could be inhibited.

We have now found that lower fatty acid lactylates are able to prevent and suppress the growth of gram-positive and gram-negative organisms in the mouth.

Oral infections/diseases are a major public health problem caused by pathogenic oral bacteria. Caries is a particular serious problem in childhood. Periodontal diseases regularly occur more frequently in adults. Oral bacteria are also the cause of tartar formation, halitosis and peri-implant infections. Only a few agents have been shown to be effective in preventing oral diseases in humans. The antifungal and bactericidal properties of lipids, fatty acids and fatty acid derivatives have been the subject of intensive study for a long time. These compounds are not only effective antiseptics but most of them are also Generally Regarded as Safe (GRAS), food approved and biodegradable. Although they are active against a wide range of bacterial species the Gram-positive species are generally more susceptible than the Gram-negative species.

This invention relates to the unknown effects of low fatty acid lactylates on the growth of oral bacteria, which are a special group of Gram-negative and Gram-positive bacteria. These organisms are commonly found in the oral cavity and are implicated in dental plaque formation and oral diseases.

Dental plaque is commonly known as the primary cause of dental caries and other oral infections. Additionally, the majority of the adults has at least minor gingivitis, an inflammation of the gums, with a much smaller percentage suffering from moderate to severe periodontitis. Dental plaque, which exists not only on the tooth surface but also under the gums, can be defined as a diverse community of microorganisms in the form of a biofilm. The microorganisms bind tightly to one another, in addition to the solid tooth surface, by means of an extracellular matrix consisting of polymers of both host and microbial origin

The presence of Gram-positive bacteria such as Streptococcus mutans is the main cause of caries, whereas Gram-negative bacteria are the cause of periodontal disease and gingivitis.

This invention pertains to an oral care product comprising a lactylate of formula I: 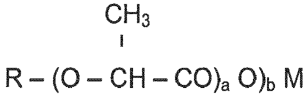 where R = CH₃(CH₂)ₙCO and n is an integer from 4 to 14, M is H⁺, an alkali metal ion, an earth alkali metal ion, or a transition metal ion; b is an integer which corresponds to the valence of M, and a is 1 or 2 , and optionally a surfactant which is not a lactylate of formula I.

The term "low fatty acid" or "low fatty acid ester" as used in this invention relates to group R wherein n is 4-14.

It was found that the preferred chain length for group R is 8 to 16, which corresponds to n = 6-14. More preferred is n = 8-12. Even more preferred is n = 10

The above lactylates are found to be effective anti-oral bacterial compounds and for that reason another objective of this invention is their use as an anti-oral bacterial compound in an oral care product.

In one embodiment the invention also relates to a method for treating and preventing oral cavity infections by orally applying the above described lactylate.

In another embodiment the invention relates to a method for preventing, reducing, controlling, and/or removing dental plaque by orally applying the above lactylate.

In yet another embodiment the invention relates to an oral care product comprising the above lactylates which are toothpastes, mouth waters, and chewing gums.

In another embodiment the products according to the invention are used to prevent or treat caries, periodontal disease, gingivitis, tartar formation, halitosis and peri-implant infections.

In another embodiment the invention relates to a method for preventing, reducing, controlling, and/or removing dental plaque by orally applying the above lactylate wherein the oral application of the lactylate is performed in the form of using a toothpaste, a mouth water, impregnated dental flosses or a chewing gum.

In yet another embodiment the invention relates to the use of toothpastes, mouth waters, and chewing gums comprising the above lactylates in preventing, reducing, controlling, and/or removing dental plaque.

With the term "mouth waters" is meant liquid preparations specifically designed to cleanse and refresh the mouth. The term "dental floss", as used herein, is defined to include dental flosses, dental tapes, and similar articles.

With the term "tooth paste" is meant any composition for cleaning, whitening, and preserving the teeth including the tooth gels and tooth powders.

The amount of lactylates in these products is between 0,05 and 10% by weight of the final product. A more preferred range is between 0,1 and 1%. Most preferred is an amount of 0,2% by weight of lactylate.

The oral care products according to the present invention are effective in inhibiting and/or killing pathogenic oral bacteria.

The oral care products may contain other common ingredients as well such as surfactants.

A surfactant can be used in the preparation of composition of the present invention to aid in the dispersion of the composition throughout the oral cavity when applied thereto as well as to improve the cosmetic acceptability and detersive and foaming properties of the composition. Among the organic surfactants useful in the practice of the present invention are salts of the higher alkyl sulfates, such as sodium lauryl sulfate (SLS) or other suitable alkyl sulfate having 8 to 18 carbon atoms in the alkyl group; sodium lauryl sulfoacetate, salts of sulfonated monoglycerides of higher fatty acids, such as sodium coconut monoglyceride sulfonate or other suitable sulfonated monoglycerides of a fatty acids of 10 to 18 carbon atoms; salts of amides of higher fatty acid, e.g., 12 to 16 carbon atom acids, with lower aliphatic amino acids, such as sodium-N-methyl-N-palmitoyl tauride, sodium N-lauroyl-, N-myristoyl- and N-palmitoyl sarcosinates; salts of the esters of such fatty acids with isothionic acid or with glycerol monosulfate, such as the sodium salt of monosulfated monoglyceride of hydrogenated coconut oil fatty acids; salts of olefin sulfonates, e.g. alkene sulfonates or hydroxalkene sulfonates or mixtures thereof having 12 to 16 carbon atoms in the carbon chain of the molecule; and soaps of higher fatty acids, such as those of 12 to 18 carbon atoms, e.g., coconut fatty acids. The cation of the salt may be sodium potassium or mono-, di or triethanol amine. Lactylates of formula I are excluded in the definition for surfactant. These compounds are only added as antimicrobial agent and have no or negligible surfactant effects.

If toothpaste is used polishing agents are incorporated in dentifrice composition of the present invention and preferred polishing agents are siliceous materials, such as silica, and will normally have a mean particle size up to about 10 microns and a very high surface area, e.g. in the range of 150-750 square meters/gram. A preferred silica is a precipitated amorphous hydrated silica but other polishing agents may also be employed, including sodium bicarbonate, calcium carbonate, sodium metaphosphate, potassium metaphosphate, tricalcium phosphate, calcium phosphate dihydrate, anhydrous dicalcium phosphate, calcium pyrophosphate, magnesium orthophosphate, trimagnesium phosphate, alumina trihydrate, aluminum silicate, zirconium silicate, calcined alumina and bentonite.

Toothpastes may further contain organic and inorganic thickeners such as fumed silicas such as Cab-o-sil available from Cabot Corporation, and thickening silicas including those available from W. R. Grace designated Sylox 15.

Organic thickeners include natural and synthetic gums. Examples of such thickeners include carrageenan (Irish moss), xanthan gum and sodium carboxymethyl cellulose, starch, polyvinylpyrrolidone, hydroxyethylpropylcellulose, hydroxybutyl methyl cellulose, hydroxypropyl methyl cellulose, and hydroxyethyl cellulose.

Peroxide compounds may be used as an ingredient in the composition of the present invention and when added to the composition are present at a concentration of about 0.25 to about 5% by weight and preferably about 0.5 to about 2.0% by weight. Peroxide compounds suitable for use in the practice of the present invention include metal peroxides such as calcium peroxide, magnesium peroxide and zinc peroxide.

Fluoride-providing salts having anti-caries efficacy may also be incorporated in the compositions of the present invention and are characterized by their ability to release fluoride ions in water. It is preferable to employ a water-soluble salt fluoride providing about 10-2000 ppm of fluoride ion, and preferably about 1000-1500 ppm of fluoride ion. Among these materials are water-soluble inorganic metal salts, for example, sodium fluoride, potassium fluoride, sodium monofluorophosphate, and sodium fluorosilicate. Sodium fluoride and sodium monofluorophosphate are preferred fluoride-providing salts.

Pyrophosphate salts having anticalculus efficacy may be used including water soluble salts such as dialkali or tetra-alkali metal pyrophosphate salts. Polyphosphate salts include the water soluble alkali metal tripolyphosphates such as sodium tripolyphosphate and potassium tripolyphosphate.

Colorants such as pigments and dyes may also be used. Pigments include non-toxic, water insoluble inorganic pigments such as titanium dioxide and chromium oxide greens, ultramarine blues and pinks and ferric oxides as well as water insoluble dye lakes prepared by extending calcium or aluminum salts of FD&C dyes on alumina such as FD&C Green #1 lake, FD&C Blue #2 lake, FD&C R&D #30 lake and FD&C # Yellow 15 lake. The pigments have a particle size in the range of 5-1000 microns, preferably 250-500 microns, and are present at a concentration of 0.5 to 3% by weight.

Dyes used in the practice of the present invention are generally food color additives presently certified under the Food Drug & Cosmetic Act for use in food and ingested drugs, including dyes such as FD&C Red No. 3 (sodium salt of tetraiodofluorescein), FD&C Yellow No. 5 (sodium salt of 4-p-sulfophenylazo-1-p-sulfophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-m ethylene}-[1-(N-ethyl-N-p-sulfobenzyl)-AE-3,5-cyclohexadienimine], FD&C Blue No. 1 (disodium salt of dibenzyldiethyldiaminotriphenylcarbinol trisulfonic acid anhydrite), FD&C Blue No. 2(sodium salt of disulfonic acid of indigotin) and mixtures thereof in various proportions.

Any suitable flavoring or sweetening material may also be incorporated in the composition of the present invention.

Examples of suitable flavoring constituents are flavoring oils, e.g., oils of spearmint, peppermint, wintergreen, sassafras, clove, sage, eucalyptus, marjoram, cinnamon, lemon, and orange, and methyl salicylate. Suitable sweetening agents include sucrose, lactose, maltose, sorbitol, xylitol, sodium cyclamate, perillartine, and sodium saccharin. Suitably, flavor and sweetening agents may together comprise from 0.01% to 5% or more of the preparations.

Various other materials may be incorporated into the composition of this invention. Non-limiting examples thereof include preservatives, silicones and chlorophyll compounds, further antibacterial agents such as chlorohexidine, halogenated diphenyl ethers such as Triclosan, desensitizing agents such as potassium nitrate and potassium citrate and mixtures thereof. These adjuvants are incorporated in the gel or paste in amounts which do not substantially adversely affect the properties and characteristics desired.

The lactylate of the invention can also be processed in chewing gum formulations. Chewing gum basically comprises a tasteless gum base, sweeteners, flavors, active ingredients and functional ingredients, including texture-regulating agents and colors. In a typical example of chewing gum, bulk sweeteners represent about 60% of the product, gum base about 30%, flavors 3-4%, functional ingredients about 3%, softeners about 2% and high intensity sweeteners (e.g., aspartame) generally about 0.5%. The most commonly used bulk sweeteners for sugar products are sucrose and dextrose. For sugar-free chewing gum, polyols such as sorbitol, xylitol, isomalt and maltitol are mainly employed. The gum base is the part that is not dissolved during chewing. Its composition of resins, elastomers, fillers, waxes, fats and emulsifiers determines the texture, flavor release and taste longevity of the final gum product. When the active ingredients are released from the gum, they are efficiently distributed throughout the mouth during the process of extended chewing. The sustained release of such ingredients from chewing gum means that their oral residence time is much longer than when released using other oral delivery methods such as dentifrices and mouthwashes. This longer residence time is likely to prolong the effect of the lactylate on oral flora.

Other common ingredients can also be added to the gum, such as ingredients that contribute to dental health, tooth whitening and fresh breath. For example, carbamide may be used to neutralize plaque acids in order to prevent caries, baking soda or calcium carbonate may be used for its well known teeth- whitening property, and zinc acetate or green tea may be added for breath-freshening purposes. Mouthwashes usually will comprise surface active agents as described herabove, flavouring oils and sweetening materials, alcohol en propylene glycol, fluoride providing salts and other bacterial growth inhibiting compounds like e.g. benzoate.

The invention is further illustrated by the following non-limitative examples.

### Legends to Figures

Fig 1. Effect of Pationic 122A (black (top) line) and Pationic 138C (grey (lower) line) on the maximum specific growth rate of Streptococcus mutans UA159 in Brain Heart Infusion broth at pH 6 and 30 C. Growth rate was measured with increments of 0.001% lactylate.
Fig 2. Effect of glyceryl-mono/di-caprylate on the maximum specific growth rate of Streptococcus mutans UA159 in Brain Heart Infusion broth at pH 6 and 30 C. Growth rate was measured with increments of 0.05% lactylate.

### Example 1

### Test materials

The following materials were received from American Ingredients Company (AIC, Kansas, USA): the sodium lactate esters of decanoic and dodecanoic acid mixture (Pationic 122A) and dodecanoic and tetradecanoic acid mixture (Pationic 138C) and glyceryl mono/di caprylate (Caprylic mono/di, Sample ID: 423704, CAS [502-54-5] for glyceryl 1 monocaprylate, [26402-26-6] generally for the monoester).

### Bacterial cultures and culture conditions

Two clinical isolates were used in this study: Streptococcus mutans C180-2 and Streptococcus mutans UA159 (ATCC 700610). Both strains were a gift from Prof.W.Crielaard (The Academic Centre for Dentistry in Amsterdam (ACTA), Amsterdam, Netherlands). Strain UA159 was originally isolated from a child with active caries, furthermore the genome of this strain has recently been sequenced. All cultures were transferred daily in screw capped tubes (100 x 16 mm) containing 10 ml brain heart infusion broth (Oxoid CM0225, Basingstoke, UK) Cultures were incubated at 30 °C without agitation.

### Culture media for the Bioscreen-C

Brain heart infusion broth was prepared with varying amounts of lactylates or glyceryl mono/di caprylate. The pH of the media was adjusted to 6.0 with 9 M sulfuric acid using a Handylab pH 12 pH meter equipped with a Blueline 16 pH (micro) probe (no. 285129163). All media were sterilized by filtration using 0.45 µm cellulose acetate filters (Minisart syringe filter, sterile and non-pyrogenic, no. 16555, Sartorius, Göttingen, Germany). 300 µl of each medium was transferred to a panel of a sterile Bioscreen honeycomb 100 well plate (Thermo electron Oy, Vantaa, Finland). Completed well plates were stored at 4 °C until further use.

### Bioscreen growth experiments

Well plates were inoculated with 3 µl of the appropriate test culture using a sterile Hamilton repeating dispenser (Hamilton, Bonaduz, Switserland). The growth rate of the test organisms was determined at 30 °C with the Bioscreen C culture system (Oy Growth Curves AB Ltd, Helsinki, Finland). The Bioscreen C kinetically measures the development of turbidity by vertical photometry in up to 200 wells simultaneously. The optical density of the cultures was automatically measured at fixed time intervals at 420 - 580 nm using a wide band filter.

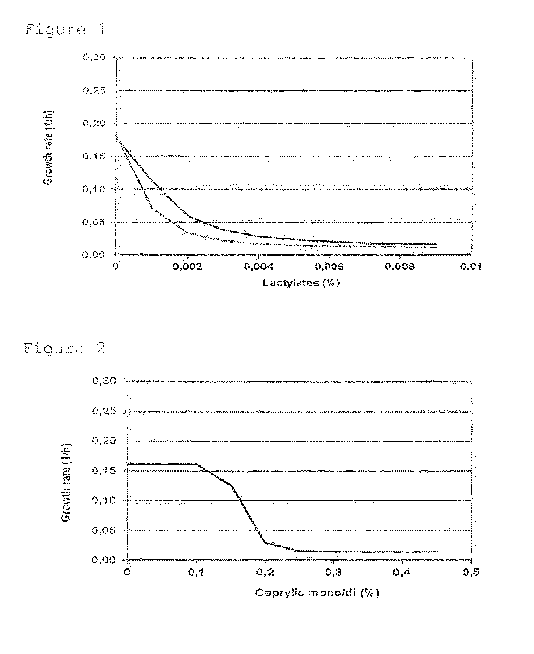

The experimental results show that all three compounds can inhibit the growth of these two clinical isolates. The results are indicated in Figures 1 and 2 and indicate that the concentration of the lactylates required for a 50% reduction in the maximum specific growth-rate is more than two orders of magnitude lower than is required for glyceryl mono/dicaprylate.

### Example 2

### Bacterial cultures and culture conditions

The following cultures were used in this study: Agregatibacter actinomycetemcomitans (HG90), Porphyromonas gingivalis (HG66), Porphyromonas gigivalis (A118, capsule mutant), and Porphyromonas gingivalis (HG 91 K-, no capsule). All strains were a gift from Prof. W.Crielaard (The Academic Center for Dentistry in Amsterdam (ACTA), Amsterdam, Netherlands).

All cultures were transferred daily in screw-capped tubes (100 x 16 mm) containing 10 ml Brain Heart Infusion broth (Oxoid CM0225, Basingstoke, UK), fortified with 1g yeast extract and 10 mL of Haemin-Menadione solution. Cultures were incubated at 37 °C without agitation.

### Test materials

The following materials were received from Caravan Ingredients company (Kansas, USA): the sodium lactate esters of octanoic acid (C8-lactylate) decanoic acid (C10-lactylate), decanoic and dodecanoic acid mixture (Pationic 122A) dodecanoic acid (C12-lactylate), dodecanoic and tetradecanoic acid mixture (Pationic 138C) tetradecanoic acid (C14-lactylate), hexadecanoic acid (C16- lactylate), oleic acid (C18:1-lactylate, Olacta).

### Culture media for the Bioscreen-C

Growth medium (37g Brain and Heart Infusion, 1 g of Yeast Extract, 10 mL of Haemin-Menadione solution, 990 mL of demi-water) was prepared with increasing amounts of the different test compounds. The concentrations ranged from 0 to 0.5% (0.05 steps) for C8 lactylate, from 0 to 0.1% (0.01 steps) for C10 lactylate, C16 lactylate and C18.1 lactylate, and from 0 to 0.01% (0.001% steps) for Pationic 122A, Pationic 138C, C12 lactylate and C14 lactylate. After preparing the media, the pH of each medium was adjusted to pH 7 with 5 M sodium hydroxide using a Handylab pH 12 pH meter equipped with a Blueline 16 pH (micro) probe (no. 285129163) and a PT1000 temperature probe W5791NN (no. 285105262) (Schott-Geräte GmbH, Mainz, Germany). All media were prepared in 10 mL quantities and sterilized by filtration. 300 µL of each medium were transferred to a panel of a sterile Bioscreen honeycomb 100 well plate (Oy Growth Curves AB Ltd, Helsinki, Finland).

### Bioscreen growth experiments

Well plates were inoculated with 3-6 µL of the appropriate test culture using a sterile Hamilton repeating dispenser (Hamilton, Bonaduz, Switserland). Growth was monitored with a Bioscreen C (Oy Growth Curves AB Ltd, Helsinki, Finland) that kinetically measures the development of turbidity by vertical photometry in up to 200 wells simultaneously. The plates were incubated at 37 °C for 16 - 24 hours under anaerobic conditions and the optical density of the cultures was measured every 30 minutes at 420 - 580 nm using a wide band filter.

Optical density values are recorded by Research Express 1.06 software for the Bioscreen C microbiology reader (Transgalactic Ltd, Helsinki, Finland). The results are expressed in terms of Minimum Inhibitory Concentrations (MIC), or minimum concentrations at which no growth (increase in OD) is observed.

The results show (Table 1) that this group of gram-negative organisms is very sensitive to the antimicrobial properties of lactylates.

**TABLE 1: Minimum Inhibitory Concentrations (MIC) of different fatty acid derivatives for four strains of toothplaque bacteria: Agregatibacter actinomycetemcomitans (HG90), Porphyromonas gingivalis (HG66), Porphyromonas gigivalis (A118) and Porphyromonas gingivalis (HG 91)**

| | **Test organism MIC values (%)** | | | |
|---|---|---|---|---|
| **Chain length** | **AA HG90** | **PG HG66** | **PG A118** | **PG HG91** |
| C8 | 0.117 | 0.45 | 0.375 | 0.15 |
| C10 | 0.02 | 0.077 | 0.055 | 0.025 |
| C10-C12 | 0.006 | 0.009 | 0.009 | 0.005 |
| C12 | 0.003 | 0.0077 | 0.005 | 0.003 |
| C12-C14 | 0.005 | 0.006 | 0.0045 | 0.0025 |
| C14 | 0.006 | 0.003 | 0.002 | 0.001 |
| C16 | >0.03 | 0.013 | 0.01 | 0.01 |

### Experiment 3

### Biofilm test

The sodium esters of C10, C12 and C14 lactylates were tested on their effect on TSP biofilm formation. TSP-biofilm is an actively attached biofilm of S.mutans grown on the pegs of a Transferable Solid Phase lid which can be used as a lid for a microtiter plate. The pegs are emerged in a liquid solution in the microtiter wells allowing the bacteria to grow on the pegs of the lid.

To this aid Streptococcus mutans strain C180-2 was used to prepare an o/n culture in BHI broth at anaerobic conditions and 37 degrees C. Thereafter a fresh inoculum was prepared by diluting the overnight culture in a 1:50 ratio to 1/2BHI + 50mM PIPES + 0.2% sucrose. 200 µL of this inoculum was dispensed in the wells of a microtiter plate. The TSP lid was placed on the microtiter plate and the and incubated in a anaerobic jar during 8 hours at 37 °C. After 8 hrs medium was refreshed and incubation was continued for an additional 16 h.

The biofilms were then washed 3 times by transferring the TSP lid to microtiter plates filled with buffered peptone water. The lids were then incubated with different amounts of active compounds and controls for 0-60 minutes in accordance with the schedule in Table 2.

After the indicated incubation time pegs were cut of the lid, transferred into 1 ml cysteine peptone water (CPW) and placed on ice. The samples were sonicated 30 times for 1 sec. at an amplitude of 40 Hz. Thereafter 100 µL of the sample was used to prepare serial dilutions in CPW. The dilutions were plated on BHI agar and incubated for 2 days at 37 °C under anaerobic conditions.

The results for C10 lactylates are shown in Table 2. Similar results were obtained for C12 lactylate which showed even after a 1 minute treatment already no detectable bacteria. Results also showed that C14 lactylates were less active than C10 and C12 lactylates.

The results of the experiment shows that the lactylates not only inhibit bacterial growth but also kill the bacteria in a biofilm.

**TABLE 2: Effect of treatment of 0.2% decanoic acid lactylate (C10) on TSP Biofilm. As a control water and Corsodyl (SmithKline Beecham, 0.2% chlorhexidine (CHX)) were applied for 5 minutes. Dl means detection limit i.e. in the range tested no bacteria were detected.**

| **Treatment** | **Treatment time (Min)** | **Average** | **STDev** |
|---|---|---|---|
| 0.2% C10 | 1 | 1.0E+05 | 1.2E+05 |
| | 5 | dl | dl |
| | 10 | dl | dl |
| | 15 | dl | dl |
| | 30 | dl | dl |
| | 60 | dl | dl |
| Milli Q | 5 | 5.7E+07 | 1.5E+07 |
| Corsodyl (0.2% CHX) | 5 | 3.3E+07 | 1.8E+07 |
| none | 0 | 3.5E+07 | 6.0E+06 |

## Claims

1. An oral care product comprising a lactylate of
formula I: 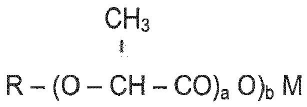 where R = CH₃(CH₂)ₙCO and n is an integer from 4 to 14, M is H⁺, an alkali metal ion, an earth alkali metal ion, or a transition metal ion; b is an integer which corresponds to the valence of M, and a is 1 or 2 , and optionally a surfactant which is not a lactylate having formula I.

2. The oral care product of claim 1 wherein n is 8 to 12.

3. The oral care product of claim 2 wherein n is 10.

4. The oral care product of claims 1-3 in the form of mouth water, toothpaste, impregnated dental flosses or chewing gum.

5. A lactylate of formula I: 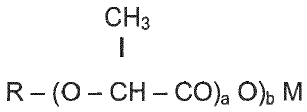 where R = CH₃(CH₂)ₙCO and n is an integer from 4 to 14, M is H⁺, an alkali metal ion, an earth alkali metal ion, or a transition metal ion; b is an integer which corresponds to the valence of M, and a is 1 or 2, for use in an oral care product.

6. The compound according to claim 5 for the prevention or treating of caries, periodontal disease, gingivitis, tartar formation, halitosis and peri-implant infections.

7. A method for treating and preventing oral cavity bacterial infections by orally applying a lactylate of formula I: 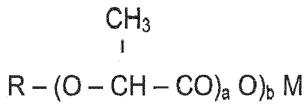 where R = CH₃(CH₂)ₙCO and n is an integer from 4 to 14, M is H⁺, an alkali metal ion, an earth alkali metal ion, or a transition metal ion; b is an integer which corresponds to the valence of M, and a is 1 or 2

8. A method for preventing, reducing, controlling and/or removing dental plaque by orally applying the oral care product of claims 1-4.

9. A method for the prevention or treating of caries, periodontal disease, gingivitis, tartar formation, halitosis and peri-implant infections by applying the oral care product of claims 1-4
